# EUROPEAN PATENT SPECIFICATION

(11) **EP 1 328 263 B1**
(45) Date of publication and mention of the grant of the patent: **15.07.2009**
(21) Application number: 01987665.5
(22) Date of filing: 18.10.2001
(51) Int. Cl.: A61K 31/12, A61K 41/00, A61P 17/00, A61P 35/00

(54) **PROCESS OF MAKING AND METHOD OF USE OF TETRAHYDROCURCUMINOIDS TO REGULATE PHYSIOLOGICAL AND PATHOLOGICAL EVENTS IN THE SKIN AND MUCOSA CELLS**
HERSTELLUNGSVERFAHREN UND VERWENDUNG VON TETRAHYDROCURCUMINOIDEN ZUR REGULIERUNG DER PHYSIOLOGISCHEN UND PATHOLOGISCHEN FÄLLE IN DER HAUT UND IN DEN SCHLEIMHAUTZELLEN
PROCEDE DE FABRICATION ET PROCEDE D'UTILISATION DE TETRAHYDROCURCUMINOIDES POUR LA REGULATION DE COMPLICATIONS PHYSIOLOGIQUES ET PATHOLOGIQUES DANS LA PEAU ET DANS LES CELLULES DES MUQUEUSES

(30) Priority: 19.10.2000 US 241364 P; 09.10.2001 US 972150
(43) Date of publication of application: 23.07.2003
(73) Proprietor: Sabinsa Corporation, Piscataway, NJ 08854 (US)
(72) Inventor: MAJEED, Muhammed, Piscataway, NJ 08854 (US); BADMAEV, Vladimir, Piscataway, NJ 08854 (US)
(74) Representative: Savic, Bojan
(86) International application number: PCT/US2001/032441
(87) International publication number: WO 2002/032415

(56) References cited:
- WO-A-00/61162
- WO-A-97/03674
- WO-A-99/55352
- T.OSAWA E.A.: "Antioxidative activity of tetrahydrocurcuminoids" BIOSCIENCE, BIOTECHNOLOGY AND BIOCHEMISTRY, vol. 59, no. 9, 1995, pages 1609-1612, XP001024945
- DATABASE CA [Online] CHEMICAL ABSTRACTS SERVICE, COLUMBUS, OHIO, US; T.OSAWA ET AL.: "Mechanisms of cancer prevention by monitoring oxidative stress" Database accession no. 130:148078 XP002223205 & KANKYO HEN'IGEN KENKYU, vol. 19, no. 3, 1997, pages 157-161,
- DATABASE WPI Week 199423, 1994 Derwent Publications Ltd., London, GB; AN 1994-188874 XP002223206 KOBE STEEL: "External agent for preventing UV damage and rough skin-contains tetrahydro-curcumin prepared by reducing curcumin extracted from turmeric root" & JP 06 128133 A (KOBE STEEL), 10 May 1994 (1994-05-10)
- S.VENKATESWARLU E.A.: "Synthesis and antibacterial activity of tetrahydrocurcuminoids" ASIAN JOURNAL OF CHEMISTRY, vol. 12, no. 1, 2000, pages 141-144, XP008011209
- T.OSAWA E.A.: "Antioxidative activity of tetrahydrocurcumin" INTERNATIONAL CONGRESS SERIES, vol. 998, 1992, pages 801-804, XP000933940

## Description

### Background of the invention

A body cell owes its functioning in large part to its cytoskeleton, the structural framework of the cell. The cytoskeleton is formed by components in the cytoplasm that maintain cell shape, stabilize cell attachments to neighboring cells, and facilitate specialized cell functions like endocytosis, exocytosis and cell motility. The cytoskeleton includes several filamentous structures like microtubules, microfilaments, intermediate filaments, and microtrabecular lattice. Proteins participating in the formation of a cytoskeleton, especially microtubular-associated proteins (MAPS) after being manufactured by the cell, are further adapted to the cytoskeleton function in a process called post-translational modification, or cross-linking. This process of proteins cross-linking is essential to stabilizing the cytoskeleton, the event necessary to maintain cell homeostasis, physiological processes, and cell development and maturation.

The post-translational modification of proteins involves several chemical reactions. Protein phosphorylation exemplifies one of the most common mechanisms of post translation modification of cellular proteins. Other examples include methylestrification of proteins, ADP-ribosylation, protein glycosylation, histone acetylation, hypusine formation and transglutaminase-catalyzed reactions.

The electric potential of a cell is an aspect of cellular physiology important to the understanding of the mechanism of the invention. The electric potential of a cell is maintained by a complex balance between negatively and positively charged molecules which results in a physiologic resting potential inside the cell of approximately - 70 mV This value is indicative of the cell's well-being. On the other hand, the inability of a cell to maintain an optimal resting potential occurs in pathological conditions, e.g. physical or chemical assaults, a disease process, and the wear and tear due to aging. Optimal resting potential is conductive to the physiological processes including post-translational protein modification and cross-linking. It also holds true that the intact physiological cross-linking of proteins is indicative of a cell's ability to maintain optimal resting electric potential,

### Positive effects of cross-linking

Cross-linking of proteins, as it has been previously mentioned, is an outcome of the post translational modification of proteins. Some of the most common cross-links identified in proteins forming cytoskeleton are Neee-(ggg-glutamyl)lysine and N,N-bis(gggglutarnyl)polyamine. The formation of these two types of cross-links is facilitated by the catalytic activity of a group of enzymes known as transglutaminases. One of the well known transglutaminases in the body is coagulation protein Factor XIII. This Factor causes cross-linking of the fibrin, which acts as a stabilizing agent on blood clot formation. This type of modification is an example of a life saving mechanism-based on protein cross-linking, that prevent exsanguination in a cut or a wound.

Transglutaminases are responsible for facilitating a physiological process of protein cross-linking, which under normal conditions leads to cell differentiation, thus the cell is able to fulfill its purpose, e.g. mucus secreting cell, phagocytic cell, epithelial and tegumen cell protecting the tissue structure. Transglutaminase activity, or its absence, plays a critical role in the differentiation of cancer cells, especially skin cancer melanoma.- Melanoma is a rapidly spreading cancer consisting of undifferentiated skin cells, and the degree of poor cell differentiation correlates well with the suppressed activity of transglutaminase. On the other hand, experimental induction of transglutaminase activity led to promising results in slowing-down or reversing melanoma progression. Examples of transglutaminase inducers are retinoic acid and methylxantines. It appears that the therapeutic potential of retinoids in melanoma is related to their ability to activate intracellular transglutaminases.

### Detrimental cross-linking

Cross-linking of intracellular proteins is, however, not always a positive event. When a random and massive cross-linking is precipitated by an abrupt, non-physiological stimulus, an accelerated aging of the cell occurs. Duc to the nature of random crosslinking, which defies the purpose of cell organization and differentiation, this may lead not only to premature cell aging and death, but also can lead to a pathology including malignancy. For example, the UVA-generated free radicals in skin cells can cause premature cross-linking of proteins and liberation of proteolytic enzymes. This latter event leads to the destruction of the cytoskeleton, and the rapid deterioration of cell organization which compromises cell functioning.

It is of special interest to mention here the term lipofuscin, nicknamed "the aging pigment" or "liver spots", which occurs in the epidermis and mucous membranes probably as a result of disrupted cellular and systemic metabolism. Lipofuscin has been shown to result from the oxidative degeneration of mitochondria and/or lysosomes in the cells. Lipofuscin is postulated to be a result of a metabolic error which results in hypoanabolism combined with hyperkatabolism. It is postulated here that lipofuscin formation is in part a manifestation of the disrupted process of post-translational protein modification, and that this process intensifies with any systemic disease process, including the aging process.

### Related art

As previously mentioned, transglutaminases are responsible for facilitating the physiological process of protein cross-linking, which under normal conditions leads to cell differentiation, maturation and timely aging. There is considerable evidence that intracellular transglutaminase activity may be inhibited in certain pathology, for example malignancy, e.g. melanoma. It appears that therapeutic potential of transglutaminase inducers like retinoids and methylxantines in melanoma is related to their ability to activate intracellular transglutaminases. This in turn would lead to differentiation of cells, which in clinical practice would lead to a slow down of the disease process due to a decrease in the number of undifferentiated malignant cells.

The role of curcuminoids, yellow phenolic compounds derived from turmeric roots (*Curcuma longa,* fam. Zingiberaceae), as topical antioxidants has been traditionally known in Asia and recently validated in laboratory experiments. Curcuminoids are reported to protect normal human keratinocytes from hypoxanthine/xanthine oxidase injury in *in vitro* studies, and they can protect the skin against a broad range of physical, chemical and biological factors injuring the skin¹. Free radicals on the surface of the skin, generated through exposure to ultraviolet radiation, especially the UVB rays, chemicals or other environmental stress factors catalyze aging of the skin. Curcuminoids prevent free radical formation and scavenge free radicals in biological systems. This combined action was previously described as a Bioprotectant mechanism that protects the integrity of the living cell e.g. skin cell. The antioxidant effects of curcuminoids combined with their known inhibitory effects on cyclooxygenase 2 (Cox-2) render them useful as ingredients in anti-aging formulations and in topical formulations designed to maintain general skin health and integrity. Curcuminoids have also been found to inhibit the activity of tyrosinase, an enzyme that participates in melanogenesis, thereby preventing melanin formation with resultant lightening of the skin tone . ²

Tetrahydrocurcuminoids (THC) are color-free compounds derived from curcuminoids, the yellow, parent compounds of THC, in the process of hydrogenation. The process of hydrogenation of curcuminoids can also occur naturally in the gastrointestinal tract.³ The tetrahydrocurcuminoids' similar biological properties to curcuminoids combined with the lack of yellow color, render them useful in achromatic food and cosmetic applications that currently employ conventional synthetic antioxidants.

Like the curcuminoids, THC have also shown significant antioxidant action in a number of *in vitro* and preclinical studies. ⁴⁻⁹ THC are valued as the ultimate metabolites of the curcuminoids *in vivo.* Several independent studies validated the significant antioxidant effects of the tetrahydrocurcuminoids and protection of skin against free radicals and UVB rays.

Curcumin derivatives including tetrahydrocurcumin and curcuminoids are well recognized in literature as a group of phenolic antioxidants (Majeed et al. Curcuminoid book). Majeed et al., U.S. Patent No. 5,861,415 established that the combination of naturally occurring curcuminoids isolated from turmeric root, i.e. curcumin, demethoxy curcumin and bisdemethoxy curcumin produce a broad range of antioxidant protection described as bioprotectant action. Bioprotectant action, characterized by the prevention of free radical formation and intervention in scavenging free radicals, is exerted by tetrahydrocurcumin(oids) (THC) as well as curcumin(oids) (Majeed et al., Tumeric and the Healing Curcuminoids, Keats Publications 1996 and Majeed et al., Curcuminoids Antioxidant Phytonutrients, NutriScience Publishers 1995). However, tetrahydrocurcumin(oids) show superior free radical scavenging properties, as compared to curcumin or curcuminoids (Majeed et al. Curcuminoid book). Another advantage of tetrahydrocurcuminoids is that they are not as highly colored curcuminoids and thus can be applied topically without significant staining.

Recently curcumin but not tetrahydrocurcumin was found to have a preventive effect on cross-linking of collagen in diabetic rats. (Sajithlal GB, Chithra P, Chandrakasan G., Biochem Pharmacol 1998 Dec 15;56(12):1607-14). However, the anti cross-linking effect of curcumin was attained with a very high oral dose of the compound (200 mg/kg body wt.). It was also noted that the preventive effect of curcumin on the advanced cross-linking of collagen was more pronounced than its therapeutic effect. The authors of this study explained that the anti cross-linking mechanism of curcumin is due to quenching the free radicals, which in turn would prevent the accelerated cross-linking of collagen in diabetes. This study supports the use of curcumin to prevent and to some degree treat excessive cross-linking of proteins in conditions characterized by accelerated cross-linking such as diabetes.

### Description of the invention

Applicants have postulated the "cross-regulin" mechanism of THC. This mechanism has been described as both qualitatively and quantitatively different from the prior art. In the experimental work leading to the present application Applicant has evaluated THC in the *in vivo* model of skin injury by the chemical agent, TPA, and the physical agent, UVB rays. In addition, Applicants have tested the practical results of post-translational modification of proteins with the invention (i.e. its effect on enzyme tyrosinrase, the enzyme which participates in melanogenesis).

### Effect of cross-regulin on TPA induced inflammation

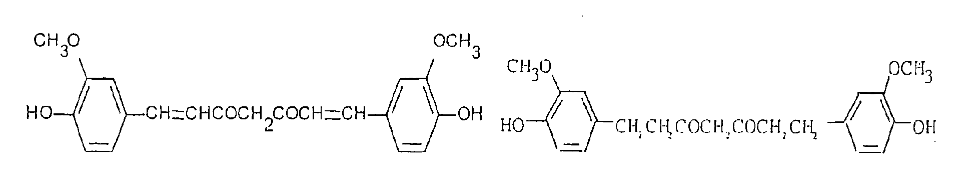

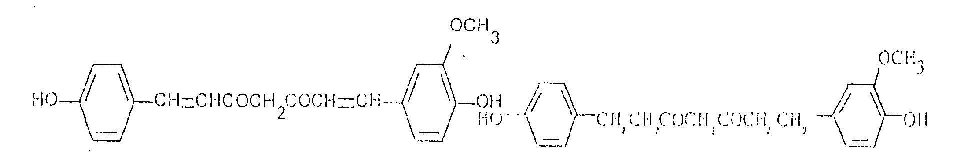

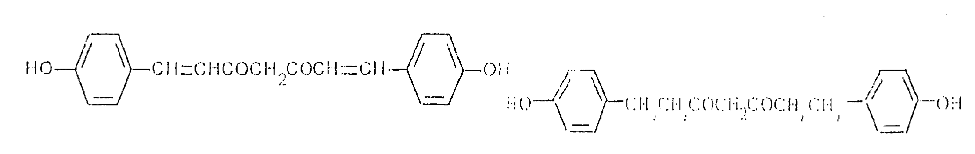

WO 00/61162 describes the method of preparation of the mixture of tetrahydrocurcuminoids according to the present invention and its use to present surburns.

TPA induces many biochemical and morphological changes in mouse skin, which appear to be associated with inflammation and skin tumor promotion. For example, topical application of TPA to the skin of mice rapidly induced skin example, topical application of TPA to the skin of mice rapidly induced skin inflammation, increased epidermal ornithine decarboxylase activity, increased epidermal DNA synthesis, and increased the number of epidermal cell layers. Ornithine decarboxylase (ODC), the first enzyme in polyamine biosynthesis, is highly regulated by many trophic stimuli, and changes in its levels and organization correlate with cytoskeletal changes in human keratinocytes. Therefore, compounds which inhibit TPA and affect ODC synthesis will also affect the organization of the cytoskeleton and related events of post-translatational modification of proteins and crosslinking of proteins in the cytosol.

Prior art teaches that chemicals that inhibit TPA-induced biochemical and morphological changes usually inhibit TPA-induced tumor promotion in mouse skin. Applicants have postulated that the *crossregulin* composition of the invention is an *optimal* composition for protecting and preserving healthy living cells and for preventing cytoskeletons of the healthy living cells from being damaged by TPA and TPA-like tumor promoters. In an experiment conducted *in vivo,* the ear skin of Female CF-1 mice was treated with either 20 microliters acetone, TPA in acetone or TPA with Tetrahydrocurcuminoids of the invention [*THC1*]*,* known Tetrahydrocurcuminoids *[THC2]* and known pure (synthetic) Tetrahydrocurcumin [*THC3*]*.*

Five hours later the mice were sacrificed and ear punches were weighted (the higher the weight, score the more severe the inflammation of the treated ear-skin). Based on the weight score of the ear punches, the percent of inflammation inhibition was calculated. Protection against inflammation in decreasing order was furnished by *THC1* of the invention, followed by *THC2* of the prior art, and finally followed by *THC3.* The protective effect was dose-dependent concerning *THC1* and *THC2,* while *THC3* showed less protection at a dose of 0.36 mg compared to a dose of 0.12 mg. Prior art, as exemplified by the research data, cannot accomplish inhibition of the TPA induced ear edema above 80%. More than 80% inhibition of TPA-induced ear edema is necessary to prevent crosslinking of proteins which could trigger skin cancer. Therefore crossregulin action is not an inherent part of just any composition of THCs, but it is a specific composition that affords inhibition of the TPA mechanism by more than 80%.

Another mechanism of the invention is in preserving homeostasis and functioning of cells based on its regulatory, both inhibitory and stimulating, effect on the cross-linking of intracellular proteins. This regulatory mechanism, previously unknown for curcumin and its derivatives, is also unrelated to the well researched and described antioxidant properties of curcumin(oids) and tetrahydrocurcumin(oids). It is a novel way of accomplishing a broad regulatory effect on biological processes. A comparable regulatory effect on cross-linking of proteins in the organism was not described previously with any single compound or with compounded products. In essence the invention differs from other compounds affecting the cross-linking process in that the previously described compounds would stimulate or inhibit intracellular cross-linking, while the invention would act as a cross-linking modifier (CLM) or "crossregulin". These terms are used here for the first time to describe the mechanism of the invention. CLM or crossregulin indicates that a composition of the present invention exerts a positive influence on the post-translational protein modification and cross-linking process in both physiological and pathological conditions alike. The CLM effect of the invention is accomplished by using a specific composition of the product as described in the section "Process of Obtaining THC". The mechanism also occurs with modifications of the THC molecule, for example ether THC, trimethyl ether THC, dimethyl ether THC, benzyl ether THC and potassium, magnesium and copper salts of THC. The mechanism of the invention has been demonstrated in *in vivo* experimental conditions such as preventing UV sunburn and cross-linking (percent of thymine dimer cells) in the skin cells exposed to UV radiation. The biological properties of the invention were compared with those of curcuminoids, and despite the fact that the present composition is devoid of yellow color it still produces comparable protective effects against UV radiation as that of yellow curcuminoids. This activity appears to be different from the antioxidant effects characteristic of THC and curcuminoids.

TPA induces many biochemical and morphological changes in mouse skin, which appear to be associated with skin tumor promotion. For example, topical application of TPA to the skin of mice rapidly induced skin inflammation, increased epidermal ornithine decarboxylase activity, increased epidermal DNA synthesis, and increased the number of epidermal cell layers. Ornithine decarboxylase (ODC), the first enzyme in polyamine biosynthesis, is highly regulated by many trophic stimuli, and changes in its levels and organization correlate with cytoskeletal changes in normal human cells including epidermal keratinocytes [Pomidor MM, Cimildoro R, Lazatin B, Zheng P, Gurr JA, Leigh IM, Janne OA, Tuan RS, Hickok NJ Phosphorylated human keratinocyte ornithine decarboxylase is preferentially associated with insoluble cellular proteins. Mol Biol Cell 1999 Dec; 10(12):4299-310.]. Therefore compounds which will inhibit TPA and affect ODC synthesis will also affect the organization of the cytoskeleton and related events of posttranslational modification of proteins and crosslinking of proteins in the cytosol.

The prior art teaches that chemicals that inhibit the TPA-induced biochemical and morphological changes usually inhibit TPA-induced tumor promotion in mouse skin. We have postulated that crossregulin composition of invention is an optimal composition for protecting and preserving healthy live cell and its cytoskeleton from being damaged by TPA and TPA-like tumor promoters. In an experiment carried *in vivo* the ear skin of Female CF-1 mice was treated with either 20 ul acetone, TPA in acetone or TPA with THcurcuminoids of the invention [*THC1*]*,* THcurcuminoids of the prior art [*THC2*] and pure Thcurcumin [*THC3*] of the prior art. Five hours later the mice were sacrificed and ear punches were weighted, the higher the weight score the more severe the inflammation of the treated ear-skin. Based on the weight score of the ear punches the percent of inflammation inhibition was calculated. The most protection against inflammation was accomplished with THC of the invention followed by THcurcuminoids of the prior art followed by Thcurcumin of the prior art. The protective effect was dose dependent with THcurcuminoids, while Thcurcumin showed less protection at a dose of 0.36 mg than 0.12 mg.

### Definition of crossregulin's action:

Based on this experimental data, *crossregulin's* action is based on an anti-inflammatory and cancer preventive mechanism which is limited to any compound that inhibits the inflammatory process by at least 80% in two tested doses (i.e. 0.12 mg and 0.36 mg).

**Table 1. Effects of THC on TPA-induced ear edema**

| Treatment | No. mice | Wg/punch | %inhibition |
|---|---|---|---|
| 1. Acetone | 2 | 6.3 ± 0.1 | - |
| 2. TPA (1nmol) | 5 | 14.2 ± 0.6 | - |
| 3. TPA THC1 0.12 | 4 | 7.7 ± 0.2 | 82.3 |
| 4. TPA THC1 0.36 mg | 4 | 7.2 ± 0.2 | 88.6 |
| | | | |
| 5. TPA THC2 0.12 mg | 4 | 9.6 ± 0.5 | 58.2 |
| 6. TPA THC2 0.36 mg | 4 | 8.3 ± 0.4 | 74.7 |
| 7. TPA THC3 0.12 mg | 4 | 7.8 ± 0.3 | 81.0 |
| 8. TPA THC3 0.36 mg | 4 | 8.1 ± 0.3 | 77.2 |

Female CF-1 mice were treated topically with 20 ul acetone, TPA (1 nmol) in acetone or TPA (1 nmol) and test compound in acetone on both ears. Five hours later the mice were sacrificed and ear punches were weighted. Data are expressed as the mean SE

THC1- invention, THC2 - prior art Mimura,et al., US patent 5,266,344 issued Nov. 30, 1993, THC3 - Pure (synthetic) tetrahydrocurcumin.

Transglutaminases form a family of proteins that have evolved for specialized functions such as protein crosslinking or posttranslational modification of proteins. In healthy tissues transglutaminases activation and the subsequent protein crosslinking is part of a protective cellular response contributing to tissue homeostasis (e.g. blood clotting, replacement of old cells to give room for a new body cells). Crosslinking has been suggested as one of the mechanisms involved in the aging process. Among the various random or enzyme-mediated crosslinking reactions, - transglutaminase induced croslinking activity has been proposed for its possible involvement in cell proliferation, differentiation and programmed death.

In case of a disease state these enzymes have been implicated in a number of pathological conditions including fibrosis, atherosclerosis, neurodegenerative diseases, celiac disease, and neoplastic disease. For example activation of transglutaminases in Alzheimer's disease may be responsible for protein crosslinking leading to deposition of amyloid material in the neural cells. The posttranslational modification or crosslinking of proteins may play an important role in both enhancement and inhibition of the initiation, promotion and spread of cancer in the body. Transglutaminases exhibit true multifunctionality at the molecular level. The crosslinking activity can subserve disparate biological phenomena depending on the stage of tumor development. For example, the ras proteins (oncogenes) can be viewed as molecular switches which when crosslinked can initiate human tumors. In certain types of skin moles oncogenes can undergo conversion to melanoma when UVA rays will trigger crosslinking of ras proteins. In that case inhibition of crosslinking will prevent initiation and progression of the malignancy.

On the other hand, activation of tissue transglutaminases in poorly differentiated metastatic cancer cell may give rise to crosslinked protein resulting in apoptosis or cancerous cell death. In this example crosslinking of proteins would have a positive impact on the clinical course of the neoplastic disease. In case of cervix carcinomas the findings indicate that specific transglutaminases are upregulated as compared to normal cervix tissue. During rat brain carcinogenesis induced by transplacental administration of N-ethyl-N-nitrosourea, the transglutaminase activity was also increased as compared to the control animals.

The invention proposes a practical solution of regulating functions of transglutaminases in both states, health and disease to the advantage treated human or animal organism. The mechanism of crossregulin is unique to a composition of the invention. Prior art, as exemplified by the research data, can not accomplish inhibition of the TPA induced ear edema above the 80%. More than 80% inhibition of TPA induced ear edema is necessary to prevent crosslinking of proteins which could trigger skin cancer. Therefore crossregulin action is not an inherent part of any composition of THCs, but a specific composition that affords inhibition of TPA mechanism by more than 80%. The composition of the invention complies with the definition of crossregulin action.

The TPA inhibition experiment shows that even doubling the dose of THCs of the prior art would not provide the above 80% inhibition, whereas THCs of the invention can afford the above 80% inhibition with either of the experimental dose. Further, the single compound THC of the prior art shows less inhibition of TPA with the increase of the experimental dose, that is unlike the composition of the invention which provides dose dependent inhibitory effect.

The importance of a minimum biological efficacy of any compound needed to display desired biological effect can not be underestimated. The anti-inflammatory mechanism is different from the anticancer mechanism and both mechanism can be related to a specific level of biological potency.

### The following describes mechanisms of this invention in detail:

1. It is proposed that in physiologic states characterized by optimal post-translational protein modification, protein cross-linking, as well as the optimal cell electric potential, THC would not interfere with the above exemplified states of cellular well-being.
2. However, in the states, characterized by poor morphological and functional differentiation of cells, e.g. pre-malignant states or malignancy, THC would contribute to protein post-translational modification and cross-linking and optimization of cell electric potential. This would be accomplished by interaction between phenolic rings and olefinic bonds of THC and NH2 as well as COO groups of the protein amino acids. The resulting bonds would act as an "anchor" that would initiate cross-linking with other proteins.
3. In the states of cell aging and in the states where random cross-linking is precipitated by a thermal injury due to the action of UV rays, for example, the undesired process would be prevented by the invention. THC would prevent undesired cross-linking by attaching to amino and carboxy groups of the protein amino acids. This would result in interference with the random cross-linking, a mechanism comparable to a "buffering" action. This "buffering" action would effectively stop the chain reaction which otherwise would lead to deterioration of cell homeostasis, premature aging and premature cell death.
4. The invention also has a supportive mechanism in preventing random cross-linking of intracellular proteins which is based on the antioxidant properties of THC, specifically on the invention's ability to scavenge free-radicals. It is proposed that due to this antioxidant action it would facilitate disruption of the electric charges of "supercharged" protein molecules, which is one of the reasons why random cross-linking is accelerated. This combined mechanism of crossregulin and antioxidant action is particularly applicable in preventing
   inflammatory conditions such as psoriasis.
5. The source of oxygen moieties in THC for binding to NH2 or COO groups would be from the para hydroxy group no longer in conjugation with the olefinic bond, and from the hydroxy groups of phenolic rings.
6. Unlike the curcumin(oid) compound(s), THC molecule(s) can rotate more freely, thus providing better access to the reactive groups for "anchor" and/or "buffering" reactions.
7. Whereas, as previously mentioned, the invention is relatively inactive in states of cell physiology, its crossregulin mechanism would be gradually expressed proportionate to the degree of stress acting upon the cell. Thus the crossregulin mechanism of THC in the biological system would depend on the kind of injurious action experienced by the biological system.

### Process of obtaining THC:

Tetrahydro curcuminoids of the invention are products of the chemical reduction of curcuminoids, obtained from the rhizomes of *Curcuma longa,* commonly known as turmeric, or any other suitable plant from the botanical family Zingiberaceae.

The prior art teaches that when curcuminoids obtained from *Curcuma longa* are catalytically reduced and then isolated in the process of crystallization, tetrahydrocurcumin (THC) is recovered without a loss, but with substantial losses of tetrahydrobisdemethoxy curcumin (THBDC) and tetrahydrodemethoxy curcumin (THDC). As a result, the ratio among tetrahydrocurcuminoids is significantly changed as compared to tile original ratio of parent compounds, curcuminoids. The ratio of curcuminoids is preferred, because it correlates with the optimal biological activity of curcuminoids. U.S. Patent No. 5,861,415 describes curcuminoids in a specific and optimal ratio as Bioprotectants, providing maximal protective effect of the living cell.

The present invention provides a new, more efficient method of recovering THCs. This process prevents the loss of TBDMC and TDMC, and provides a Bioprotectant composition of THCs.

| THCurcuminoids | % content prior art | % content invention |
|---|---|---|
| Tetrahydro curcuminoids | 90 | 75 - 85 |
| Tetrahydro demethoxy curcumin | 9 | 10 - 20 |
| Tetrahydro bisdemethoxy curcumin | 1 | 2 - 4.5 |

### Brief outline of the prior art process for producing THCs

| | |
|---|---|
| Natural curcuminoids (95 %) | - 100 gm |
| Acetone | - 1 liter |
| Pd-C (catalyst) 5% | - 2.5 gm |

The solution of natural curcuminoids was charged into a hydrogenator. The catalyst was added carefully and the pressure of hydrogen was maintained at about 2 Kg/cm2. This reaction is completed in 3 to 4 hrs. The catalyst is then filtered out and removed. The acetone solution is concentrated under vacuum. The residue is crystallized from toluenes. The tetrahydro compounds are crystallized out as a creamy white powder having a melting point of 92-94°C.

### Process of the Present invention

This process utilizes a novel method of saturation of two olefinic bonds in curcuminoids in the catalytic hydrogen transfer reaction known as a hydride transfer reaction. The following is an example of a hydride transfer method.
1. A 3 liter round bottom flask with a stirrer and a reflux condenser is charged with 1 liter ethyl acetate;
2. The solution is charged with 250 gm of natural curcuminoids in a previously described Bioprotectant composition, i.e. curcumin (C) 70-80%, Demethoxy curcumin (DMC) 15-20% and Bisdemethoxy curcumin (BDMC 2.5-6.5% and stirred to uniformity at room temperature;
3. This mixture is charged with 1 liter of triethylamine followed by 12 gm of Palladium carbon and 80 ml of formic acid;
4. The mixture was stirred and subsequently refluxed at 80 deg. Celsius for 12 hours.
5. Periodically formic acid in 5 ml aliquots was charged at 2 hours intervals while a reflux was carried on;
6. TLC was checked to monitor the completion of the reaction. (system: Silica Precoated 0.25 mm thick plates/chloroform : methanol = 9: 1);
7. After completion of the reaction, the reaction mixture was cooled to room temperature. It was filtered to remove palladium catalyst and washed with 50 ml of ethyl acetate;
8. Ethyl acetate and triethylamine were distilled off (2 liters of distillate was collected);
9. The crude mass was then extracted with toluene (3 x 500 ml);
10. The toluene extract was washed with 200 ml of 5% HCI and 3 x 500 ml of water and dried over sodium sulphate;
11. The solvent was removed under vacuum to get a pale yellow paste approximately 200 gm.
12. The paste was slurred with 200 ml of ether, filtered and dried under vacuum to obtain a pale yellow powder of tetrahydrocurcuminoids, Yield 175 gm (70%).
Assay:

| | |
|---|---|
| THC | 75 to 85% |
| THDHC | 10 to 20% |
| THBDMC | 2 to 4.5% |

In another variation of hydride transfer reaction, curcuminoids were treated with ammonium formate or sodium dihydrogen phosphite (as a catalytic hydrogen transfer reagent) in the presence of Pd/C and acetic acid. The reduction is complete in 20 to 30 minutes at 100 to 110 Celsius degrees. This method yields 50% of tetrahydrocurcuminoids.

THC can be administered topically in a suitable vehicle or with an application device, e.g. a transdermal patch, in a concentration ranging from 0.025% to 5%; in the form of subcutaneous injections or a transdermal patch in a concentration ranging from 0.025% to 5%; or in orally administered dosage form in a concentration ranging from 5 mg to 500 mg per dose or 0.083 - 8.3 mg/kg of body weight. THC is suitable for human and animal use.

Other routes of administration include but are not limited to: aerosol or other device for delivery to the lungs, nasal spray, intravenous, intramuscular, intraperitoneal, intrathecal, vaginal, and rectal. Excipients which can be used as a vehicle for the delivery of the phage will be apparent to those skilled in the art.

Compositions of tetrahydrocurcuminoids for topical administration should be essentially colorless. The term "essentially colorless" is intended to mean that the composition contains no color or such a small amount of color that the composition does not stain the skin upon topical administration.

The term "color removed curcumin" refers to the tetrahydrocurcuminoid mixture according to the present invention.

THC molecule acting as an "anchor" for amino acids of proteins, to initiate protein cross-links. This mechanism would contribute to the physiological cross-links.

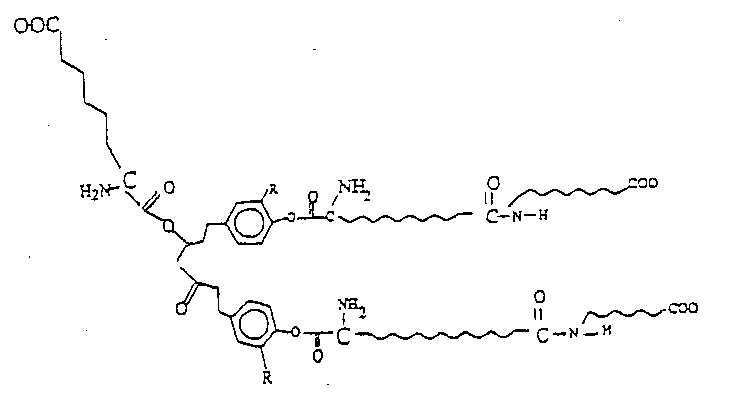

Legend: 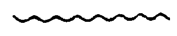 amino acid/protein

THC molecule acting as a buffer in preventing random cross-links in UV precipitated cross-links. This mechanism would contribute to the prevention of pathological cross-links.

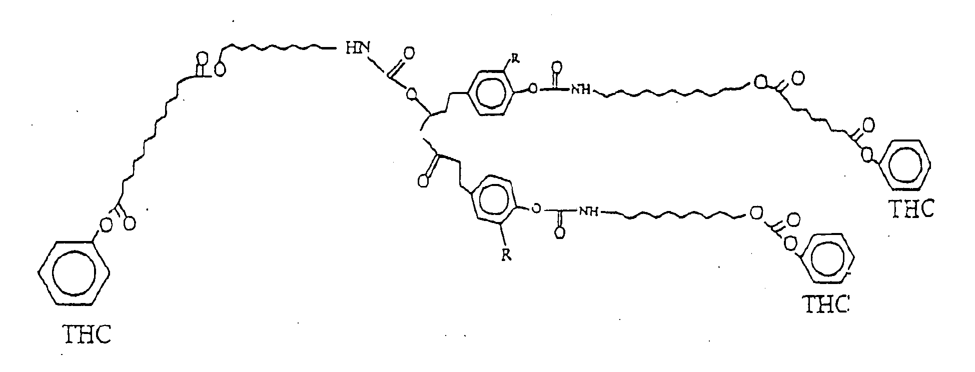

Legend: 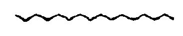 amino acid/protein

### References

1. Majeed, M. et al. (1995) Curcuminoids: Antioxidant Phytonutrients. Nutriscience Publishers, New Jersey.
2. Shirota et al. (1994) Tyrosinase inhibitors from crude drugs. Biol Pharm Bull 17(2):266-269
3. Pan, M.H. et al. (1999) Biotransformation of curcumin through reduction and glucuronidation in mice. Drug Metab. Dispos. 27(4):486-94.
4. Osawa, T. et al. (1995) Antioxidative activity of the tetrahydrocurcuminoids. Biosci. Biotechnol. Biochem. 59(9): 1609-12.
5. Sugiyama, Y. (1996) Involvement of the beta-diketone moiety in the antioxidative mechanism of Tetrahydrocurcumin. Biochem Pharmacol, 52(4):519-25 Aug 23
6. Nakamura, Y. et al. (1998) Inhibitory effects of curcumin and tetrahydrocurcuminoids on the tumor-promoter-induced reactive oxygen species generation in leukocytes, in vitro and in vivo.. Jpn J Cancer Res, 89(4):361-70
7. Mukhopadhaya, A. et al. (1982). Anti-inflammatory and irritant activities of curcumin analogues in rats, Agents and Action. 12,2287.
8. Rao, T.S.,_ et al. (1982) Antiinflammatory activity of curcumin analogues. Ind J. Med.Res., 75 574-578
9. Bont'e, F. et al. (1997) Protective effects of curcuminoids on epidermal skin cells under free oxygen radical stress. Planta Med. 63(3):265-266.
10. Mimura,et al., US patent 5,266,344 issued Nov. 30, 1993
11. Sakuma et al. (1999 Aug.). Relationship between tyrosinase inhibitory action and oxidation-reduction potential of cosmetic whitening ingredients and phenol derivatives. Arch Pharm Res. 1999. 22(4):335-339).
12. Kollias N, Sayre RM, Zeise L, Chedekel MR. Photoprotection by melanin. J Photochem Photobiol B 1991 May;9(2):135-160
13. Morison WL. Effects of ultraviolet radiation on the immune system in humans. Photochem Photobiol 1989 Oct;50(4):515-524.
14. Li W, Hill HZ. Induced melanin reduces mutations and cell killing in mouse melanoma. Photochem Photobiol 1997 Mar; 65(3):480-485.

## Claims

1. A mixture comprising :
75-85% tetrahydrocurcumin ;
10-20% tetrahydrodemethoxy curcumin; and
2,0-4,5% tetrahydrobisdemethoxy curcumin
For use in preventing inflammatory conditions, with a proviso that the inflammatory condition is not UV-sunburn,

## Patentansprüche

1. Eine Mischung umfassend:
75-85 % Tetrahydrocurcumin;
10-20 % Tetrahydrodemethoxycurcumin; und
2,0 - 4,5 % Tetrahydrobisdemethoxycurcumin zur Verwendung
als Verhütung entzündlicher Zustände mit der Maßgabe, daß der entzündliche Zustand kein UV-Sonnenbrand ist.

## Revendications

1. Composition comprenant :
75-85% de tétrahydrocurcumine;
10-20% de tétrahydrodéméthoxycurcumine;
2,0-4,5% de tétrahydrobisdéméthoxycurcumine;
pour l'utilisation en prévention d'états inflammatoires, sous réserve de ce que l'état inflammatoire ne soit pas une brûlure due au rayonnement UV du soleil.
